(19) Europäisches Patentamt
European Patent Office
Office européen des brevets

(11) EP 1 447 442 A1

(12) # EUROPEAN PATENT APPLICATION

(43) Date of publication:
**18.08.2004 Bulletin 2004/34**

(51) Int Cl.[7]: **C12N 1/20**, C12P 19/40

(21) Application number: **04003190.8**

(22) Date of filing: **12.02.2004**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **17.02.2003 JP 2003037760**

(71) Applicant: **Ajinomoto Co., Inc.**
**Tokyo 104-8315 (JP)**

(72) Inventors:
- **Tominaga, Misa**
  **Morotomi-Machi Saga-gun Saga (JP)**
- **Shimazaki, Keishi**
  **Morotomi-Machi Saga-gun Saga (JP)**
- **Matsuno, Kiyoshi**
  **Kawasaki-ku Kawasaki-shi Kanagawa (JP)**
- **Yamashita, Minoru**
  **Kawasaki-ku Kawasaki-shi Kanagawa (JP)**

(74) Representative: **Strehl Schübel-Hopf & Partner**
**Maximilianstrasse 54**
**80538 München (DE)**

(54) **Inosine production by means of Bacillus bacteria able to grow in the presence of 6-ethoxypurine**

(57) Strains showing favorable growth resistance in a medium containing 6-ethoxypurine are selected from a population of *Bacillus* bacteria, and a strain showing high inosine-producing ability is selected from the obtained strains to obtain a *Bacillus* bacterium having improved inosine-producing ability.

EP 1 447 442 A1

SEQUENCE LISTING

<110> Ajinomoto Co., Inc.

<120> Inosine producing bacterium belonging to the genus Bacillus and method for producing inosine

<130> EPA-63377

<150> JP 2003-37760
<151> 2003-02-17

<160> 16

<170> PatentIn Ver. 2.0

<210> 1
<211> 28
<212> DNA
<213> Artificial Sequence

<220>
<223> Description of Artificial Sequence: primer

<400> 1
ctcaagcttg aagttgcgat gatcaaaa 28

<210> 2
<211> 28
<212> DNA
<213> Artificial Sequence

<220>
<223> Description of Artificial Sequence: primer

<400> 2
ctcctgcaga catattgttg acgataat 28

<210> 3
<211> 29
<212> DNA

<213> Artificial Sequence

<220>
<223> Description of Artificial Sequence: primer

<400> 3
ctcgtcgaca aaacgaatgg aagcgaacg 29

<210> 4
<211> 29
<212> DNA
<213> Artificial Sequence

<220>
<223> Description of Artificial Sequence: primer

<400> 4
ctcgcatgca gaccaactta tatgcggct 29

<210> 5
<211> 29
<212> DNA
<213> Artificial Sequence

<220>
<223> Description of Artificial Sequence: primer

<400> 5
ctcgaattcc agcggaatat tctttcccg 29

<210> 6
<211> 28
<212> DNA
<213> Artificial Sequence

<220>
<223> Description of Artificial Sequence: primer

<400> 6
ctcggatccc ggcaaaagca cagtatcc 28

<210> 7
<211> 30
<212> DNA
<213> Artificial Sequence

<220>
<223> Description of Artificial Sequence: primer

<400> 7
ctcaagctta tggtttccag accatcgact 30

<210> 8
<211> 28
<212> DNA
<213> Artificial Sequence

<220>
<223> Description of Artificial Sequence: primer

<400> 8
ctcggatccc atgatatgat agaagtgg 28

<210> 9
<211> 33
<212> DNA
<213> Artificial Sequence

<220>
<223> Description of Artificial Sequence: primer

<400> 9
ctcggatccg aggtgatagg taagattata ccg 33

<210> 10
<211> 33
<212> DNA
<213> Artificial Sequence

<220>
<223> Description of Artificial Sequence: primer

<400> 10
ctcggatccg gcgctcggga cccctatcta gcg 33

<210> 11
<211> 1200
<212> DNA
<213> Bacillus subtilis

<220>
<221> CDS
<222> (259)..(1113)

<400> 11
atatgcatcc tgaagttgcg atgatcaaaa accagatgaa acgctttggt gcagatgccg 60
tgttaatgag cgggagcggc ccgacagtgt ttggactggt tcagtatgag tcgaaggtgc 120
agagaattta taacgggtta agaggcttct gcgatcaagt ttatgcggtg agaatgatcg 180
gcgaacagaa cgctcttgat taaatccgta tgttaagtta tattgatctt aaaatattcg 240
gattttgggg gtgagttc atg aag ttt cgt cgc agc ggc aga ttg gtg gac 291
Met Lys Phe Arg Arg Ser Gly Arg Leu Val Asp
1 5 10
tta aca aat tat ttg tta acc cat ccg cac gag tta ata ccg cta acc 339
Leu Thr Asn Tyr Leu Leu Thr His Pro His Glu Leu Ile Pro Leu Thr
15 20 25
ttt ttc tct gag cgg tat gaa tct gca aaa tca tcg atc agt gaa gat 387
Phe Phe Ser Glu Arg Tyr Glu Ser Ala Lys Ser Ser Ile Ser Glu Asp
30 35 40
tta aca att att aaa caa acc ttt gaa cag cag ggg att ggt act ttg 435
Leu Thr Ile Ile Lys Gln Thr Phe Glu Gln Gln Gly Ile Gly Thr Leu
45 50 55
ctt act gtt ccc gga gct gcc gga ggc gtt aaa tat att ccg aaa atg 483
Leu Thr Val Pro Gly Ala Ala Gly Gly Val Lys Tyr Ile Pro Lys Met
60 65 70 75
aag cag gct gaa gct gaa gag ttt gtg cag aca ctt gga cag tcg ctg 531
Lys Gln Ala Glu Ala Glu Glu Phe Val Gln Thr Leu Gly Gln Ser Leu
80 85 90
gca aat cct gag cgt atc ctt ccg ggc ggt tat gta tat tta acg gat 579
Ala Asn Pro Glu Arg Ile Leu Pro Gly Gly Tyr Val Tyr Leu Thr Asp
95 100 105
atc tta gga aag cca tct gta ctc tcc aag gta ggg aag ctg ttt gct 627
Ile Leu Gly Lys Pro Ser Val Leu Ser Lys Val Gly Lys Leu Phe Ala
110 115 120

tcc gtg ttt gca gag cgc gaa att gat gtt gtc atg acc gtt gcc acg 675
Ser Val Phe Ala Glu Arg Glu Ile Asp Val Val Met Thr Val Ala Thr
125 130 135
aaa ggc atc cct ctt gcg tac gca gct gca agc tat ttg aat gtg cct 723
Lys Gly Ile Pro Leu Ala Tyr Ala Ala Ala Ser Tyr Leu Asn Val Pro
140 145 150 155
gtt gtg atc gtt cgt aaa gac aat aag gta aca gag ggc tcc aca gtc 771
Val Val Ile Val Arg Lys Asp Asn Lys Val Thr Glu Gly Ser Thr Val
160 165 170
agc att aat tac gtt tca ggc tcc tca aac cgc att caa aca atg tca 819
Ser Ile Asn Tyr Val Ser Gly Ser Ser Asn Arg Ile Gln Thr Met Ser
175 180 185
ctt gcg aaa aga agc atg aaa acg ggt tca aac gta ctc att att gat 867
Leu Ala Lys Arg Ser Met Lys Thr Gly Ser Asn Val Leu Ile Ile Asp
190 195 200
gac ttt atg aaa gca ggc ggc acc att aat ggt atg att aac ctg ttg 915
Asp Phe Met Lys Ala Gly Gly Thr Ile Asn Gly Met Ile Asn Leu Leu
205 210 215
gat gag ttt aac gca aat gtg gcg gga atc ggc gtc tta gtt gaa gcc 963
Asp Glu Phe Asn Ala Asn Val Ala Gly Ile Gly Val Leu Val Glu Ala
220 225 230 235
gaa gga gta gat gaa cgt ctt gtt gac gaa tat atg tca ctt ctt act 1011
Glu Gly Val Asp Glu Arg Leu Val Asp Glu Tyr Met Ser Leu Leu Thr
240 245 250
ctt tca acc atc aac atg aaa gag aag tcc att gaa att cag aat ggc 1059
Leu Ser Thr Ile Asn Met Lys Glu Lys Ser Ile Glu Ile Gln Asn Gly
255 260 265
aat ttt ctg cgt ttt ttt aaa gac aat ctt tta aag aat gga gag aca 1107
Asn Phe Leu Arg Phe Phe Lys Asp Asn Leu Leu Lys Asn Gly Glu Thr
270 275 280
gaa tca tgacaaaagc agtccacaca aaacatgccc cagcggcaat cgggccttat 1163
Glu Ser
285
tcacaaggga ttatcgtcaa caatatgttt tacagct 1200

<210> 12
<211> 285
<212> PRT
<213> Bacillus subtilis

<400> 12

Met Lys Phe Arg Arg Ser Gly Arg Leu Val Asp Leu Thr Asn Tyr Leu
1 5 10 15
Leu Thr His Pro His Glu Leu Ile Pro Leu Thr Phe Phe Ser Glu Arg
20 25 30
Tyr Glu Ser Ala Lys Ser Ser Ile Ser Glu Asp Leu Thr Ile Ile Lys
35 40 45
Gln Thr Phe Glu Gln Gln Gly Ile Gly Thr Leu Leu Thr Val Pro Gly
50 55 60
Ala Ala Gly Gly Val Lys Tyr Ile Pro Lys Met Lys Gln Ala Glu Ala
65 70 75 80
Glu Glu Phe Val Gln Thr Leu Gly Gln Ser Leu Ala Asn Pro Glu Arg
85 90 95
Ile Leu Pro Gly Gly Tyr Val Tyr Leu Thr Asp Ile Leu Gly Lys Pro
100 105 110
Ser Val Leu Ser Lys Val Gly Lys Leu Phe Ala Ser Val Phe Ala Glu
115 120 125
Arg Glu Ile Asp Val Val Met Thr Val Ala Thr Lys Gly Ile Pro Leu
130 135 140
Ala Tyr Ala Ala Ala Ser Tyr Leu Asn Val Pro Val Val Ile Val Arg
145 150 155 160
Lys Asp Asn Lys Val Thr Glu Gly Ser Thr Val Ser Ile Asn Tyr Val
165 170 175
Ser Gly Ser Ser Asn Arg Ile Gln Thr Met Ser Leu Ala Lys Arg Ser
180 185 190
Met Lys Thr Gly Ser Asn Val Leu Ile Ile Asp Asp Phe Met Lys Ala
195 200 205
Gly Gly Thr Ile Asn Gly Met Ile Asn Leu Leu Asp Glu Phe Asn Ala
210 215 220
Asn Val Ala Gly Ile Gly Val Leu Val Glu Ala Glu Gly Val Asp Glu
225 230 235 240
Arg Leu Val Asp Glu Tyr Met Ser Leu Leu Thr Leu Ser Thr Ile Asn
245 250 255
Met Lys Glu Lys Ser Ile Glu Ile Gln Asn Gly Asn Phe Leu Arg Phe
260 265 270
Phe Lys Asp Asn Leu Leu Lys Asn Gly Glu Thr Glu Ser
275 280 285

<210> 13
<211> 1490
<212> DNA
<213> Bacillus subtilis

<220>
<221> CDS
<222> (101)..(1393)

<400> 13
aattaatgtt cggatttaca attgactttc tgtttcttca ctgataaact tgatttgttt 60
gaatagaatc gttttgaaag gttaacggag gtgcacggac atg tct tca gta gtt 115
Met Ser Ser Val Val
1 5
gta gta ggt acg caa tgg ggc gat gaa gga aaa ggt aaa att aca gat 163
Val Val Gly Thr Gln Trp Gly Asp Glu Gly Lys Gly Lys Ile Thr Asp
10 15 20
ttc cta tca gaa aat gca gaa gtg atc gcc cgt tat caa ggc gga aat 211
Phe Leu Ser Glu Asn Ala Glu Val Ile Ala Arg Tyr Gln Gly Gly Asn
25 30 35
aac gca ggg cat aca atc aag ttt gac gga atc aca tat aag ctt cac 259
Asn Ala Gly His Thr Ile Lys Phe Asp Gly Ile Thr Tyr Lys Leu His
40 45 50
tta atc ccg tct gga att ttc tat aag gat aaa acg tgt gta atc gga 307
Leu Ile Pro Ser Gly Ile Phe Tyr Lys Asp Lys Thr Cys Val Ile Gly
55 60 65
aac gga atg gtt gta gat ccg aaa gca tta gtc aca gag ctt gcg tat 355
Asn Gly Met Val Val Asp Pro Lys Ala Leu Val Thr Glu Leu Ala Tyr
70 75 80 85
ctt cat gag cgc aac gtg agt aca gat aac ctg aga atc agc aac aga 403
Leu His Glu Arg Asn Val Ser Thr Asp Asn Leu Arg Ile Ser Asn Arg
90 95 100
gct cac gtc att ctg ccg tat cat ttg aaa ttg gat gaa gtg gaa gaa 451
Ala His Val Ile Leu Pro Tyr His Leu Lys Leu Asp Glu Val Glu Glu
105 110 115
gag cgt aaa ggg gct aac aag atc ggc aca acg aaa aaa gga atc ggc 499
Glu Arg Lys Gly Ala Asn Lys Ile Gly Thr Thr Lys Lys Gly Ile Gly
120 125 130
cct gct tac atg gat aaa gca gcc cgc atc gga att cgc atc gcg gat 547
Pro Ala Tyr Met Asp Lys Ala Ala Arg Ile Gly Ile Arg Ile Ala Asp
135 140 145
ctg tta gac cgt gac gcg ttt gcg gaa aag ctt gag cgc aat ctt gaa 595
Leu Leu Asp Arg Asp Ala Phe Ala Glu Lys Leu Glu Arg Asn Leu Glu
150 155 160 165
gaa aaa aac cgt ctt ctc gag aaa atg tac gag aca gaa ggg ttt aaa 643

Glu Lys Asn Arg Leu Leu Glu Lys Met Tyr Glu Thr Glu Gly Phe Lys
170 175 180

ctt gag gat atc tta gac gaa tat tat gag tac gga cag cag att aaa 691
Leu Glu Asp Ile Leu Asp Glu Tyr Tyr Glu Tyr Gly Gln Gln Ile Lys
185 190 195

aag tat gtt tgc gat aca tct gtt gtc tta aac gat gct ctt gat gaa 739
Lys Tyr Val Cys Asp Thr Ser Val Val Leu Asn Asp Ala Leu Asp Glu
200 205 210

ggg cgc cgt gta tta ttt gaa ggc gca caa ggg gtt atg ctc gat atc 787
Gly Arg Arg Val Leu Phe Glu Gly Ala Gln Gly Val Met Leu Asp Ile
215 220 225

gac caa gga aca tac ccg ttt gtt acg tca tct aac ccg gtt gcc ggc 835
Asp Gln Gly Thr Tyr Pro Phe Val Thr Ser Ser Asn Pro Val Ala Gly
230 235 240 245

ggt gtc acg atc ggt tct ggt gtc ggc ccg acc aaa atc aag cac gtt 883
Gly Val Thr Ile Gly Ser Gly Val Gly Pro Thr Lys Ile Lys His Val
250 255 260

gtc ggt gta tca aaa gca tat acg act cgt gtc ggc gac ggt cct ttt 931
Val Gly Val Ser Lys Ala Tyr Thr Thr Arg Val Gly Asp Gly Pro Phe
265 270 275

ccg act gag ctg aaa gat gaa atc ggc gat caa atc cgt gaa gtc gga 979
Pro Thr Glu Leu Lys Asp Glu Ile Gly Asp Gln Ile Arg Glu Val Gly
280 285 290

cgc gaa tat gga aca aca aca ggc cgc ccg cgc cgt gtc ggc tgg ttt 1027
Arg Glu Tyr Gly Thr Thr Thr Gly Arg Pro Arg Arg Val Gly Trp Phe
295 300 305

gac agc gtt gtt gtc cgc cac gcc cgc cgt gtg agc gga att aca gat 1075
Asp Ser Val Val Val Arg His Ala Arg Arg Val Ser Gly Ile Thr Asp
310 315 320 325

ctt tct ctg aac tca att gac gtc cta gca gga att gaa acg ttg aaa 1123
Leu Ser Leu Asn Ser Ile Asp Val Leu Ala Gly Ile Glu Thr Leu Lys
330 335 340

atc tgt gtg gcg tac cgc tac aaa ggc gaa atc att gaa gaa ttc cca 1171
Ile Cys Val Ala Tyr Arg Tyr Lys Gly Glu Ile Ile Glu Glu Phe Pro
345 350 355

gca agt ctt aag gca ctt gct gaa tgt gag ccg gta tat gaa gaa atg 1219
Ala Ser Leu Lys Ala Leu Ala Glu Cys Glu Pro Val Tyr Glu Glu Met
360 365 370

ccg ggc tgg act gag gat att aca ggt gcg aag agc ttg agc gag ctt 1267
Pro Gly Trp Thr Glu Asp Ile Thr Gly Ala Lys Ser Leu Ser Glu Leu
375 380 385

ccg gaa aat gcg cgc cat tat ctt gag cgt gtg tct cag ctg aca ggc 1315
Pro Glu Asn Ala Arg His Tyr Leu Glu Arg Val Ser Gln Leu Thr Gly
390 395 400 405
att ccg ctt tct att ttc tct gtc ggt cca gac cgc tca caa aca aat 1363
Ile Pro Leu Ser Ile Phe Ser Val Gly Pro Asp Arg Ser Gln Thr Asn
410 415 420
gtc ctt cgc agt gtg tac cgt gcg aac taa atagaatatg tctgcaagcc 1413
Val Leu Arg Ser Val Tyr Arg Ala Asn
425 430
cctatttaag ggcttgttt tttgtttgaa agccgcatat aagttggtct gagaaaaaaa 1473
tatgaaaaaa aaccaaa 1490

<210> 14
<211> 430
<212> PRT
<213> Bacillus subtilis

<400> 14
Met Ser Ser Val Val Val Val Gly Thr Gln Trp Gly Asp Glu Gly Lys
1 5 10 15
Gly Lys Ile Thr Asp Phe Leu Ser Glu Asn Ala Glu Val Ile Ala Arg
20 25 30
Tyr Gln Gly Gly Asn Asn Ala Gly His Thr Ile Lys Phe Asp Gly Ile
35 40 45
Thr Tyr Lys Leu His Leu Ile Pro Ser Gly Ile Phe Tyr Lys Asp Lys
50 55 60
Thr Cys Val Ile Gly Asn Gly Met Val Val Asp Pro Lys Ala Leu Val
65 70 75 80
Thr Glu Leu Ala Tyr Leu His Glu Arg Asn Val Ser Thr Asp Asn Leu
85 90 95
Arg Ile Ser Asn Arg Ala His Val Ile Leu Pro Tyr His Leu Lys Leu
100 105 110
Asp Glu Val Glu Glu Glu Arg Lys Gly Ala Asn Lys Ile Gly Thr Thr
115 120 125
Lys Lys Gly Ile Gly Pro Ala Tyr Met Asp Lys Ala Ala Arg Ile Gly
130 135 140
Ile Arg Ile Ala Asp Leu Leu Asp Arg Asp Ala Phe Ala Glu Lys Leu
145 150 155 160
Glu Arg Asn Leu Glu Glu Lys Asn Arg Leu Leu Glu Lys Met Tyr Glu
165 170 175
Thr Glu Gly Phe Lys Leu Glu Asp Ile Leu Asp Glu Tyr Tyr Glu Tyr

180 185 190
Gly Gln Gln Ile Lys Lys Tyr Val Cys Asp Thr Ser Val Val Leu Asn
195 200 205
Asp Ala Leu Asp Glu Gly Arg Arg Val Leu Phe Glu Gly Ala Gln Gly
210 215 220
Val Met Leu Asp Ile Asp Gln Gly Thr Tyr Pro Phe Val Thr Ser Ser
225 230 235 240
Asn Pro Val Ala Gly Gly Val Thr Ile Gly Ser Gly Val Gly Pro Thr
245 250 255
Lys Ile Lys His Val Val Gly Val Ser Lys Ala Tyr Thr Thr Arg Val
260 265 270
Gly Asp Gly Pro Phe Pro Thr Glu Leu Lys Asp Glu Ile Gly Asp Gln
275 280 285
Ile Arg Glu Val Gly Arg Glu Tyr Gly Thr Thr Thr Gly Arg Pro Arg
290 295 300
Arg Val Gly Trp Phe Asp Ser Val Val Val Arg His Ala Arg Arg Val
305 310 315 320
Ser Gly Ile Thr Asp Leu Ser Leu Asn Ser Ile Asp Val Leu Ala Gly
325 330 335
Ile Glu Thr Leu Lys Ile Cys Val Ala Tyr Arg Tyr Lys Gly Glu Ile
340 345 350
Ile Glu Glu Phe Pro Ala Ser Leu Lys Ala Leu Ala Glu Cys Glu Pro
355 360 365
Val Tyr Glu Glu Met Pro Gly Trp Thr Glu Asp Ile Thr Gly Ala Lys
370 375 380
Ser Leu Ser Glu Leu Pro Glu Asn Ala Arg His Tyr Leu Glu Arg Val
385 390 395 400
Ser Gln Leu Thr Gly Ile Pro Leu Ser Ile Phe Ser Val Gly Pro Asp
405 410 415
Arg Ser Gln Thr Asn Val Leu Arg Ser Val Tyr Arg Ala Asn
420 425 430

<210> 15
<211> 899
<212> DNA
<213> Bacillus subtilis

<220>
<221> CDS
<222> (101)..(802)

<400> 15

atatattcct ttataatcaa tgctgaagtt tgtttcatca tgacattcgt tgtaaaatgg 60
acgggctgga tgataattta gatttacagg aggatatgag atg agt gta cat ata 115
Met Ser Val His Ile
1 5
ggt gct gaa aaa gga caa att gcg gat act gtg ctt ttg ccg gga gat 163
Gly Ala Glu Lys Gly Gln Ile Ala Asp Thr Val Leu Leu Pro Gly Asp
10 15 20
cct ctc aga gca aaa ttt att gca gaa acg tat ctt gaa aat gta gaa 211
Pro Leu Arg Ala Lys Phe Ile Ala Glu Thr Tyr Leu Glu Asn Val Glu
25 30 35
tgc tac aat gaa gtc aga ggc atg tat gga ttt acg ggt aca tat aaa 259
Cys Tyr Asn Glu Val Arg Gly Met Tyr Gly Phe Thr Gly Thr Tyr Lys
40 45 50
ggt aaa aaa atc tca gta caa ggc acg gga atg gga gtt ccg tct att 307
Gly Lys Lys Ile Ser Val Gln Gly Thr Gly Met Gly Val Pro Ser Ile
55 60 65
tca att tat gtg aat gaa tta att caa agc tac gat gtg caa aat cta 355
Ser Ile Tyr Val Asn Glu Leu Ile Gln Ser Tyr Asp Val Gln Asn Leu
70 75 80 85
ata aga gtc ggt tcc tgc ggc gct att cgt aaa gat gtc aaa gtg cga 403
Ile Arg Val Gly Ser Cys Gly Ala Ile Arg Lys Asp Val Lys Val Arg
90 95 100
gac gtc att ttg gcg atg acc tcc tca act gat tca caa atg aac aga 451
Asp Val Ile Leu Ala Met Thr Ser Ser Thr Asp Ser Gln Met Asn Arg
105 110 115
gtt gct ttc gga agc gtt gat ttt gcg cct tgc gca gat ttc gag ctt 499
Val Ala Phe Gly Ser Val Asp Phe Ala Pro Cys Ala Asp Phe Glu Leu
120 125 130
tta aaa aat gcc tat gat gcc gca aag gat aaa ggt gtg ccg gtg act 547
Leu Lys Asn Ala Tyr Asp Ala Ala Lys Asp Lys Gly Val Pro Val Thr
135 140 145
gta gga agc gta ttt aca gct gac cag ttc tac aat gac gat tcg caa 595
Val Gly Ser Val Phe Thr Ala Asp Gln Phe Tyr Asn Asp Asp Ser Gln
150 155 160 165
att gaa aaa ctt gca aaa tac ggt gtg ctt ggc gtt gaa atg gaa aca 643
Ile Glu Lys Leu Ala Lys Tyr Gly Val Leu Gly Val Glu Met Glu Thr
170 175 180
act gca ttg tat aca tta gca gcg aag cac gga aga aaa gcc ctg tca 691
Thr Ala Leu Tyr Thr Leu Ala Ala Lys His Gly Arg Lys Ala Leu Ser
185 190 195

att ctc acc gtg agt gat cac gta tta aca gga gaa gaa acg aca gcg 739
Ile Leu Thr Val Ser Asp His Val Leu Thr Gly Glu Glu Thr Thr Ala
200 205 210
gaa gag cgt caa acg aca ttt cat gat atg ata gaa gtg gct tta cat 787
Glu Glu Arg Gln Thr Thr Phe His Asp Met Ile Glu Val Ala Leu His
215 220 225
tcc gta tca caa taa aatatatcaa gaggcgtgct gggtgccggc agcctcttct 842
Ser Val Ser Gln
230
ttatgcatgc ggaaacgatt gataaaagga aggtaatgta atatgaaaaa gtccggt 899

<210> 16
<211> 233
<212> PRT
<213> Bacillus subtilis

<400> 16
Met Ser Val His Ile Gly Ala Glu Lys Gly Gln Ile Ala Asp Thr Val
1 5 10 15
Leu Leu Pro Gly Asp Pro Leu Arg Ala Lys Phe Ile Ala Glu Thr Tyr
20 25 30
Leu Glu Asn Val Glu Cys Tyr Asn Glu Val Arg Gly Met Tyr Gly Phe
35 40 45
Thr Gly Thr Tyr Lys Gly Lys Lys Ile Ser Val Gln Gly Thr Gly Met
50 55 60
Gly Val Pro Ser Ile Ser Ile Tyr Val Asn Glu Leu Ile Gln Ser Tyr
65 70 75 80
Asp Val Gln Asn Leu Ile Arg Val Gly Ser Cys Gly Ala Ile Arg Lys
85 90 95
Asp Val Lys Val Arg Asp Val Ile Leu Ala Met Thr Ser Ser Thr Asp
100 105 110
Ser Gln Met Asn Arg Val Ala Phe Gly Ser Val Asp Phe Ala Pro Cys
115 120 125
Ala Asp Phe Glu Leu Leu Lys Asn Ala Tyr Asp Ala Ala Lys Asp Lys
130 135 140
Gly Val Pro Val Thr Val Gly Ser Val Phe Thr Ala Asp Gln Phe Tyr
145 150 155 160
Asn Asp Asp Ser Gln Ile Glu Lys Leu Ala Lys Tyr Gly Val Leu Gly
165 170 175
Val Glu Met Glu Thr Thr Ala Leu Tyr Thr Leu Ala Ala Lys His Gly
180 185 190

Arg Lys Ala Leu Ser Ile Leu Thr Val Ser Asp His Val Leu Thr Gly
195 200 205
Glu Glu Thr Thr Ala Glu Glu Arg Gln Thr Thr Phe His Asp Met Ile
210 215 220
Glu Val Ala Leu His Ser Val Ser Gln
225 230

## Description

BACKGROUND OF THE INVENTION

Field of the Invention

The present invention relates to a method for producing inosine, which is an important substance useful as a synthetic raw material to produce 5'-inosinic acid, and a novel microorganism useful for the production thereof.

Description of the Related Art

There are known methods for fermentative production of inosine utilizing microorganisms of the genus *Bacillus,* which are adenine-auxotrophic strains or adenine-auxotrophic strains further imparted with resistance to various substances, including purine analogues (Japanese Patent Publication (KOKOKU) Nos. 38-23099, 54-17033, 55-2956, 55-45199, 57-14160, 57-41915 and Japanese Patent Laid-open (KOKAI) No. 59-42895), microorganisms of the genus *Brevibacterium* (Japanese Patent Publication (KOKOKU) Nos. 51-5075, 58-17592, Agric. Biol. Chem., 42, 399 (1978)), and so forth.

In order to obtain such mutant strains, a method of performing a mutagensis treatment such as ultraviolet irradiation or nitrosoguanidine (N-methyl-N'-nitro-N-nitrosoguanidine) treatment and screeninga target mutant strain using a suitable selection medium has been conventionally used. On the other hand, breeding of nucleic acid-producing strains using genetic engineering techniques has also been performed for microorganisms of the genus *Bacillus* (Japanese Patent Laid-open (KOKAI) Nos. 58-158197, 58-175493, 59-28470, 60-156388, 1-27477, 1-174385, 3-58787, 3-164185, 5-84067, 5-192164, 11-346778 (U.S. Patent No. 6,284,495)) and microorganisms of the genus *Brevibacterium* (Japanese Patent Laid-open (KOKAI) No. 63-248394). Specifically, for example, a method of using a *Bacillus* bacterium in which the repressor protein gene *(purR)* of the purine operon was disrupted to efficiently produce nucleic acid compounds such as hypoxanthine, uracil, guanine and adenine has been disclosed (Japanese Patent Laid-open (KOKAI) No. 11-346778 (U.S. Patent No. 6,284,495)).

Moreover, for *Bacillus subtilis,* it is known that the aforementioned repressor protein controls expressions of the *purA* gene involved in the biosynthesis of adenine and genes of the pyrimidine operon involved in pyrimidine biosynthesis, in addition to the genes of the purine operon (H. Zalkin et al., J. Bacteriol., 179, 7394-7402, 1997).

Furthermore, for *Escherichia coli,* it is known that its purine nucleoside-producing ability is improved by disrupting the succinyl-AMP synthase gene *(purA)* to impart adenine auxotrophy (International Patent Publication WO99/03988). Furthermore, it is also known that the purine nucleoside-producing ability is improved by disrupting the purine nucleoside phosphorylase gene *(deoD)* to repress the degradation of inosine and guanosine into hypoxanthine and guanine (International Patent Publication WO99/03988).

SUMMARY OF THE INVENTION

An object of the present invention is to create a microorganism suitable for the production of inosine by fermentation and provide a method for producing inosine using such a microorganism.

It is a further object of the present invention to provide a *Bacillus* bacterium which is modified so that growth inhibition by 6-ethoxypurine is reduced and has inosine-producing ability.

It is a further object of the present invention to provide the *Bacillus* bacterium as described above, which is a mutant strain derived from a parent strain belonging to the genus *Bacillus* and shows favorable growth as compared with the parent strain when cultured in a medium containing 6-ethoxypurine.

It is a further object of the present invention to provide the bacterium as described above, wherein the medium has an ethoxypurine content of 2000 mg/L.

It is a further object of the present invention to provide the bacterium as described above, wherein the medium is a solid medium.

It is a further object of the present invention to provide the bacterium as described above, wherein when the bacterium is cultured by inoculationg a suspension of the bacterium to a solid medium containing 6-ethoxypurine and a solid medium not containing 6-ethoxypurine, the bacterium shows a relative growth degree of 80 or more, which is defined by the following equation:

Relative growth degree (%) = [colony diameter (mm) observed in the medium containing 6-ethoxypurine]/[colony diameter (mm) observed in the medium not containing 6-ethoxypurine] x 100

It is a further object of the present invention to provide the bacterium as described above, wherein the solid medium containing 6-ethoxypurine has a 6-ethoxypurine content of 2000 mg/L.

It is a further object of the present invention to provide the bacterium as described above, wherein the solid medium is a minimal medium.

It is a further object of the present invention to provide the bacterium as described above, which is deficient in one or more genes negatively acting on the biosynthesis of inosine or involved in degradation of inosine, and selected from a purine operon repressor gene, succinyl-AMP synthase gene and purine nucleoside phosphorylase gene.

It is a still further object of the present invention to provide a method for producing a *Bacillus* bacterium having improved inosine-producing ability, which comprises selecting strains showing favorable growth in a medium containing 6-ethoxypurine from a population of *Bacillus* bacteria and selecting a strain showing high inosine-producing ability from the obtained strains.

It is even a further object of the present invention to provide the method as described above, wherein the population of *Bacillus* bacteria is obtained by subjecting a parent strain belonging to the genus *Bacillus* to a mutagenesis treatment.

According to the present invention, a microorganism having an inosine-producing ability and a method for producing inosine using the microorganism are provided.

BRIEF DESCRIPTION OF THE DRAWINGS

Fig. 1 is a graph showing inosine (HxR) and hypoxanthine (Hyp) production amounts of a 6-ethoxypurine-resistant strain.

DESCRIPTION OF THE PREFERRED EMBODIMENTS

The inventors of the present invention conducted research in order to achieve the aforementioned objects. As a result, it was found that inosine-producing ability of a *Bacillus* bacterium could be improved by modifying the *Bacillus* bacterium so that the growth inhibition by 6-ethoxypurine is reduced, and the inventors thus accomplished the present invention.

The *Bacillus* bacterium of the present invention is modified so that the growth inhibition by 6-ethoxypurine is reduced, and it has an inosine-producing ability.

The expression "the inosine-producing ability" as used herein means an ability of the *Bacillus* bacterium of the present invention to cause accumulation of inosine in a medium in an amount sufficient to enable collection of the inosine from the medium. For example, 4 g/L or more of inosine and hypoxanthine in terms of inosine amount, when the bacterium is cultured in the medium.

Examples of the *Bacillus* bacterium include, but are not limited to, *Bacillus subtilis, Bacillus amyloliquefaciens, Bacillus pumilus* and so forth.

Examples of the *Bacillus subtilis* include, but are not limited to, the *Bacillus subtilis* 168 Marburg strain (ATCC 6051), *Bacillus subtilis* PY79 (Plasmid, 1984, 12, 1-9) and so forth, and examples of *Bacillus amyloliquefaciens* include, but are not limited to, *Bacillus amyloliquefaciens* T (ATCC 23842), *Bacillus amyloliquefaciens* N (ATCC 23845) and so forth.

The *Bacillus* bacterium of the present invention can be obtained by modifying a strain having an inosine-producing ability so that the growth inhibition by 6-ethoxypurine is reduced. The *Bacillus* bacterium of the present invention can also be obtained by modifying a strain modified so that the growth inhibition by 6-ethoxypurine is reduced so as to be imparted with an inosine-producing ability or so that its inosine-producing ability is improved.

A *Bacillus* bacterium having an inosine-producing ability can be obtained by, for example, imparting adenine auxotrophy or, in addition to the adenine auxotrophy, resistance to a drug such as a purine analogue to a *Bacillus* bacterium (Japanese Patent Publication Nos. 38-23099, 54-17033, 55-2956, 55-45199, 57-14160, 57-41915 and Japanese Patent Laid-open Publication No. 59-42895). A *Bacillus* bacterium having an auxotrophy or drug resistance as described above can be obtained by ultraviolet irradiation or a treatment with a mutagenizing agent used for typical mutagenesis treatment such as N-methyl-N'-nitro-N-nitrosoguanidine (NTG) or EMS (ethyl methanesulfonate).

Methods for breeding a *Bacillus* bacterium having an inosine-producing ability include the following examples. For example, the methods encompass increasing intracellular activity of an enzyme involved in the inosine biosynthesis, specifically, increasing expression of the gene of the aforementioned enzyme. The phrase "increasing intracellular activity" means increasing the activity to a level higher than that of a non-modified *Bacillus* bacterium, such as a wild-type *Bacillus* bacterium. Examples include, but are not limited to, increasing the number of enzyme molecules per cell, increasing specific activity per enzyme molecule and so forth.

Examples of the enzyme involved in the inosine biosynthesis include, for example, phosphoribosyl pyrophosphate (PRPP) amidotransferase, phosphoribosyl pyrophosphate (PRPP) synthetase, adenosine deaminase and so forth.

Moreover, the examples also include canceling regulation of an enzyme involved in inosine biosynthesis, specifically, a method of canceling feedback inhibition of such an enzyme (WO99/03988 Examples of the means for canceling regulation of such an enzyme as mentioned above involved in the inosine biosynthesis include, for example, deletion of the purine repressor (U.S. Patent No. 6,284,495). Examples of the method of deleting the purine repressor include disrupting a gene coding for the purine repressor *(purR,* GenBank Accession No. Z99104).

Moreover, the inosine-producing ability can also be increased by blocking a reaction branching off from the inosine biosynthesis and resulting in another metabolic product (WO99/03988). Examples of the reaction branching off from the inosine biosynthesis and resulting in another metabolic product include reactions catalyzed by, for example, succinyl-adenosine monophosphate (AMP) synthase, inosine-guanosine kinase, 6-phosphogluconate dehydrase, phosphoglucoisomerase and so forth. The succinyl-adenosine monophosphate (AMP) synthase is encoded by *purA* (GenBank Accession No. Z99104).

Furthermore, the inosine-producing ability can also be enhanced by weakening incorporation of inosine into cells. The incorporation of inosine into cells can be weakened by blocking a reaction involved in the incorporation of inosine into cells. Examples of the aforementioned reaction involved in the incorporation of the inosine into cells include a reaction catalyzed by, for example, nucleoside permease.

Furthermore, the inosine-producing ability can also be improved by reducing or eliminating inosine degradation activity (WO99/03988 Examples of the method of reducing or eliminating inosine degradation activity include a method of disrupting a gene coding for purine nucleoside phosphorylase *(deoD).*

Increasing the activity of a target enzyme in cells of a *Bacillus* bacterium can be attained by enhancing the expression of a gene coding for the enzyme.

Enhancing the expression of the gene can be attained by increasing the copy number of the gene. For example, the gene fragment coding for the aforementioned enzyme can be ligated to a vector which functions in *Bacillus* bacteria, preferably a multi-copy type vector, to prepare a recombinant DNA, which is then used to transform the host Bacillus bacterium.

As the gene to be introduced, any of genes derived from *Bacillus* bacteria and genes derived from other organisms such as *Escherichia* bacteria may be used, so long as the gene functions in the *Bacillus* bacterium.

The target gene can be obtained by, for example, PCR method (polymerase chain reaction, see White, T.J. et al., Trends Genet., 5, 185 (1989)) using a chromosomal DNA of a *Bacillus* bacterium as a template. The chromosomal DNA can be prepared from a bacterium serving as a DNA donor by, for example, the method of Saito and Miura (see H. Saito and K. Miura, Biochem. Biophys. Acta, 72, 619 (1963), Text for Bioengineering Experiments, Edited by the Society for Bioscience and Bioengineering, Japan, pp.97-98, Baifukan, 1992) or the like. The primers for PCR can be prepared based on a known gene sequence of a *Bacillus* bacterium or based on information on a region conserved among genes of other bacteria of which sequences are known, or the like.

Examples of an autonomously replicable vector for introducing a target gene into a *Bacillus* bacterium include, for example, pUB110, pC194, pE194 and so forth. Furthermore, examples of a vector for incorporating a target gene into a chromosomal DNA include vectors for *E. coli,* such as pHSG398 (Takara Shuzo) and pBluescript SK- (Stratagene).

To prepare a recombinant DNA, a target gene can be ligated with a vector which functions in a *Bacillus* bacterium by digesting the vector with a restriction enzyme suitable for the end of the target gene. The ligation is typically performed by using a ligase such as T4 DNA ligase.

To introduce the recombinant DNA prepared as described above into a *Bacillus* bacterium, any known method for transformation can be employed. Examples include, for instance, preparing competent cells from cells which are at the growth phase followed by introducing the DNA thereinto, (Dubunau and Davidoff-Abelson, J. Mol. Biol., 56, 209 (1971); Duncan, C.H., Wilson, G.A. and Young, F.E., Gene, 1, 153 (1977)) and a method of making host cells into protoplasts or spheroplasts, which can easily incorporate recombinant DNA, followed by introducing the recombinant DNA into the DNA-acceptor cells (Chang, S. and Choen, S.N., Molec. Gen. Genet., 168, 111 (1979)).

Copy number of a target gene can also be increased by allowing multiple copies of the gene to exist on chromosomal DNA of a *Bacillus* bacterium. Multiple copies of the target gene may be introduced into chromosomal DNA of a *Bacillus* bacterium by, for example, homologous recombination. This can be performed by targeting a sequence present on chromosomal DNA in multiple copy number. A repetitive DNA or an inverted repeat present at the end of a transposable element can be used as the sequence present on the chromosomal DNA in a multiple copy number.

Besides the above gene amplification methods, the activity of a target enzyme can be amplified by replacing an expression regulatory sequence, such as the promoter of the target gene on a chromosomal DNA or plasmid, with a stronger one. Moreover, it is also possible to introduce nucleotide substitution for several nucleotides into a promoter region of the target gene so that it is modified into a stronger one. Such modification of expression regulatory sequence may be combined with the increase of copy number of the target gene.

The substitution of an expression regulatory sequence can be performed, for example, in the same manner as that for the gene substitution described below.

Examples of the method for reducing an activity of a target enzyme of a *Bacillus* bacterium include treating the *Bacillus* bacterium with ultraviolet ray irradiation or a mutagenizing agent used in a typical mutagenesis treatment such as N-methyl-N'-nitro-N-nitrosoguanidine (NTG) or EMS (ethyl methanesulfonate) and selecting a mutant strain showing reduced activity of the target enzyme.

Furthermore, a *Bacillus* bacterium having a reduced activity of a target enzyme can also obtained by replacing a gene coding for the target enzyme on a chromosome with a corresponding gene that does not normally function (hereinafter, also referred to as "disrupted-type gene") by homologous recombination utilizing a gene recombination technique (Experiments in Molecular Genetics, Cold Spring Harbor Laboratory Press (1972); Matsuyama, S. & Mizushima, S., J. Bacteriol., 162, 1196 (1985)).

Substitution of a disrupted-type gene for a normal gene on a host chromosome can be performed as follows. In the following example, disruption of the *purR gene is* exemplifying. However, disruption of other genes, for example, *purA* and *deoD,* can also be similarly performed.

Homologous recombination is a phenomenon that when a plasmid, or the like, containing a sequence homologous to a chromosomal sequence is introduced into a cell, recombination results at the site in the sequence having homology at a certain frequency, and the introduced plasmid as a whole is incorporated into the chromosome. Thereafter, if further recombination results at the site in the sequence having homology on the chromosome, the plasmid is eliminated again from the chromosome. At this time, the gene introduced with a mutation may be fixed on the chromosome, and the native gene may be eliminated along with the plasmid, depending on the site where the recombination occurs.

By selecting such a strain, a strain in which the deletion type *purR* gene substitutes for the normal *purR* gene on the chromosome can be obtained.

Gene disruption by homologous recombination are known, as are methods using linear DNA, methods using a plasmid, including a temperature sensitive replication control region, and so forth. Furthermore, the *purR* gene can be disrupted by using a plasmid which contains the *purR* gene inserted with a marker gene, such as a drug resistance gene, and cannot replicate in a target microbial cell. That is, a transformant which has been transformed with such a plasmid as mentioned above and thus acquired the drug resistance is incorporated with the marker gene in the chromosomal DNA. Since it is highly probably that the marker gene is incorporated by the homologous recombination of the *purR* gene sequence at the both ends thereof, and the *purR* gene on a chromosome, a gene-disrupted strain can be efficiently selected.

The disrupted-type *purR* gene used for the gene disruption can be obtained by, specifically, using deletion of a certain region of the *purR* gene using digestion with a restriction enzyme and re-ligation, insertion of another DNA fragment (marker gene etc.) into the *purR* gene, site-directed mutagenesis (Kramer, W. and Frits, H.J., Methods in Enzymology, 154, 350 (1987)), or a treatment with a chemical agent such as sodium hyposulfite and hydroxylamine (Shortle, D. and Nathans, D., Proc. Natl. Acad. Sci. U.S.A., 75, 270 (1978)) to cause substitution, deletion, insertion, addition or inversion of one or more nucleotides in the nucleotide sequence of the coding region, promoter region or the like of the *purR* gene and thereby reduce or eliminate the activity of the encoded repressor, or reduce or eliminate the transcription of the *purR* gene. Among these methods, the method of deleting a certain region of the *purR* gene by digestion with a restriction enzyme and re-ligation or the method of inserting another DNA fragment into the *purR* gene is preferred in view of the reliability and stability.

The *purR* gene can be obtained from a chromosomal DNA of a microorganism having the purine operon by PCR using oligonucleotides prepared based on the known nucleotide sequence of the *purR* gene as primers. *The purR* gene can also be obtained from a chromosomal DNA library of a microorganism having the purine operon by the hybridization method using an oligonucleotide prepared based on the known nucleotide sequence of the *purR* gene as a probe. The nucleotide sequence of the *purR* gene was reported for the *Bacillus subtilis* 168 Marburg strain (GenBank accession No. D26185 (the coding region corresponds to the nucleotide numbers 118041 to 118898), DDBJ Accession No.Z99104 (the coding region corresponds to the nucleotide numbers 54439 to 55296)). The nucleotide sequence of the *purR* gene and the amino acid sequence encoded by the gene are shown in SEQ ID NOS: 11 and 12.

The primers used for PCR may be any of those which allow for amplification of the *purR* gene, and specific examples include oligonucleotides having the nucleotide sequences shown in SEQ ID NOS: 1 and 2.

Similarly, examples of the primers for amplification of the *purA* gene include oligonucleotides having the nucleotide sequences shown in SEQ ID NO: 3 and 4, and examples of the primers for amplification of the *deoD* gene include oligonucleotides having the nucleotide sequences shown in SEQ ID NOS: 5 to 8. The nucleotide sequences of the *purA* gene and *deoD* gene were reported as DDBJ Accession No. Z99104.

The nucleotide sequence of the *purA* gene and the amino acid sequence encoded by the gene are shown in SEQ ID NOS: 13 and 14. Furthermore, the nucleotide sequence of the *deoD* gene and the amino acid sequence encoded by the gene are shown in SEQ ID NOS: 15 and 16.

Each of the *purR, purA* and *deoD* genes used in the present invention is not necessarily required to have the full length for use in the preparation of a disrupted-type gene thereof, and it may have a length required for causing gene disruption. Furthermore, the microorganism used for obtaining each gene is not particularly limited so long as the gene has homology in such a degree that the gene causes homologous recombination with the homologous gene of the microorganism used for the creation of a gene-disrupted strain. However, it is usually preferable to use a gene derived from the same bacterium as the target *Bacillus* bacterium.

Examples of DNA that may cause homologous recombination with the *purR, purA* or *deoD* gene of a *Bacillus* bacterium include DNAs coding for an amino acid sequence represented in SEQ ID NO: 12, 14 or 16 including substitution, deletion, insertion or addition of one or several amino acid residues. The number of "several" amino acid residues mentioned above is, for example, between 2 to 50, preferably between 2 to 30, more preferably between 2 to 10.

Specific examples of DNA that can cause homologous recombination with the aforementioned *purR, purA* or *deoD* gene of *Bacillus* bacterium include DNAs having homology of, for example, 70% or more, preferably 80% or more, more preferably 90% or more, most preferably 95% or more, to the nucleotide sequence shown in SEQ ID NO: 11, 13 or 15. More specifically, the examples include DNAs hybridyzable with the nucleotide sequence shown in SEQ ID NO: 11, 13 or 15 under stringent conditions. Examples of the stringent conditions include a condition in which washing is performed at a salt concentration of 1 x SSC, 0.1% SDS, preferably 0.1 x SSC, 0.1% SDS, at 60°C.

Examples of the marker gene mentioned above include drug resistance genes such as a spectinomycinresistance gene and kanamycin-resistance gene. The spectinomycin-resistance gene of *Enterococcus faecalis* can be obtained by preparing the plasmid pDG1726 from the *Escherichia coli* ECE101 strain commercially available from the Bacillus Genetic Stock Center (BGSC) and excising the resistance gene as a cassette from the plasmid. Furthermore, the erythromycin-resistance gene of *Staphylococcus aureus* can be obtained by preparing the plasmid pDG646 from the *Escherichia coli* ECE91 strain commercially available from the Bacillus Genetic Stock Center (BGSC) and excising the resistance gene as a cassette from the plasmid. Furthermore, the kanamycinresistance gene can be obtained by performing PCR using the pDG783 plasmid containing the kanamycin-resistance gene derived from *Streptococcus faecalis* (which can be prepared from the *Escherichia coli* ECE94 strain commercially available from the Bacillus Genetic Stock Center) as a template and the primers shown in SEQ ID NOS: 9 and 10.

When a drug resistance gene is used as the marker gene, a *purR* gene-disrupted strain can be obtained by inserting the drug resistance gene into the *purR* gene at an appropriate site, transforming a microorganism with the obtained plasmid and selecting a transformant that has become drug resistant. Disruption of the *purR* gene on a chromosome can be confirmed by analyzing the *purR* gene or the marker gene on the chromosome using Southern blotting or PCR. Incorporation of the aforementioned spectinomycin-resistance gene, erythromycin-resistance gene or kanamycin-resistance gene into a chromosomal DNA can be confirmed by PCR using primers which can amplify these genes.

Hereinafter, reduction of growth inhibition in *Bacillus* bacterium by 6-ethoxypurine will be explained. If 6-ethoxypurine exists in a medium, growth of *Bacillus* bacteria is inhibited. That is, if a *Bacillus* bacterium is cultured in a medium containing 6-ethoxypurine, the bacterium would not grow or the growth becomes slower compared to when the bacterium is cultured in a medium not containing 6-ethoxypurine. Specifically, when the bacterium is cultured on a solid medium for a certain period of time, the diameter of colonies, for example, becomes smaller in a medium containing 6-ethoxypurine compared with that observed in a medium not containing 6-ethoxypurine. On the other hand, the *Bacillus* bacterium of the present invention is modified so that the aforementioned growth inhibition by 6-ethoxypurine is reduced.

In a *Bacillus* bacterium modified so that the growth inhibition by 6-ethoxypurine is reduced as described above, the inosine-producing ability is improved as compared to a non-modified strain. Therefore, the characteristic concerning 6-ethoxypurine can be applied to breeding of inosine producing bacteria *of Bacillus* bacteria. That is, a *Bacillus* bacterium having an improved inosine-producing ability can be produced by selecting strains exhibiting favorable growth in a medium containing 6-ethoxypurine from a population of *Bacillus* bacteria and selecting a strain showing high inosine-producing ability from the obtained strains.

The *Bacillus* bacterium of the present invention can be obtained as a mutant strain derived from a *Bacillus* bacterium as a parent strain. The mutagenesis treatment for obtaining such a mutant strain is not particularly limited, and examples thereof include, but are not limited to, a treatment with UV irradiation or a treatment with a mutagenizing agent used for typical mutagenesis treatment such as N-methyl-N'-nitro-N-nitrosoguanidine (NTG) and nitrous acid.

A mutant strain whereby growth inhibition by 6-ethoxypurine is reduced is selected as a strain exhibiting more favorable growth compared with a non-modified strain, for example, the parent strain, when they are cultured in a medium containing 6-ethoxypurine, for example. That is, it can be said that, in the *Bacillus* bacterium of the present invention, sensitivity to 6-ethoxypurine is reduced, or the resistance to 6-ethoxypurine is improved, compared with the parent strain.

Examples of the aforementioned medium include, but are not limited to a minimal medium. Examples of the minimal medium include, for example, a medium having the following composition: 20 g/L of glucose, 5 g/L of ammonium chloride, 4 g/L of potassium dihydrogenphosphate, 0.01 g/L off errous sulfate, 0.01 g/L of manganese sulfate, 0.5 g/L of sodium citrate, pH 7.0.

In the present invention, the minimal medium may contain nutrients indispensable for the growth, if needed. For example, many of inosine producing strains are adenine auxotrophic, and therefore adenine is added to the medium at a concentration required for the growth. However, if the amount of adenine is too large, the growth inhibition effect of a certain kind of purine analogue may be reduced, and therefore the amount of adenine is preferably limited. Specifically, a concentration of about 0.1 g/L is preferred.

The selection of a target mutant strain or evaluation of growth inhibition by 6-ethoxypurine for the obtained mutant strain can be performed either in a liquid medium or on a solid medium. When a solid medium is used, for example, the mutant strain and the parent strain are each cultured in a liquid medium until a logarithmic growth phase or stationary phase is attained, then the culture broth is diluted with the medium, sodium chloride solution or the like, the obtained cell suspension is applied to a solid medium containing 6-ethoxypurine, and the mutant strain and the parent strain are cultured under the same conditions. The culture is usually performed at a temperature around the optimum growth temperature, for example, 34°C, for one to three days. Then, if the colonies of the mutant strain that emerge are larger than those of the parent strain, the growth of the mutant strain is more favorable than that of the parent strain, and growth inhibition by 6-ethoxypurine is reduced. The amount of ethoxypurine added to the medium is, for example, 1000 mg/L or more, preferably about 2000 mg/L.

Furthermore, when a liquid medium is used, cell suspensions of the mutant strain and the parent strain are cultured and diluted in the same manner as described above, and each inoculated into a liquid medium containing 6-ethoxypurine. The cells are cultured at a temperature around the optimum growth temperature, for example, 34°C, for several hours to one day, preferably about 6 hours. The amount of ethoxypurine added to the medium is, for example, 500 mg/L or more. If the mutant strain exhibits a higher optical density (OD) or turbidity of the medium either in, at least, a logarithmic growth phase or stationary phase, as compared with the parent strain, the growth is evaluated as favorable. Specifically, if the cells more quickly reach a logarithmic growth phase, or if the maximum value of the OD is higher, then growth is more favorable. The aforementioned logarithmic growth phase refers to a period in which the number of cells logarithmically increases on the growth curve. The stationary phase refers to a period after the logarithmic growth phase has passed and where cell division and growth have ceased, and the increase in cell number is no longer seen (Dictionary of Biochemistry, 3rd Edition, Tokyo Kagaku Dojin).

When a liquid medium is used, it may be more difficult to detect the difference in growth inhibition by 6-ethoxypurine as compared with when a solid medium is used. In the present invention, even if a difference in growth inhibition by 6-ethoxypurine cannot be detected using a liquid medium, a mutant strain is a strain which exhibits favorable growth as referred to in the present invention, so long as the mutant strain exhibits more favorable growth as compared with the parent strain on a solid medium.

Furthermore, the degree of growth inhibition by 6-ethoxypurine can also be evaluated by applying a suspension of the mutant strain to a solid medium containing 6-ethoxypurine and a solid medium not containing 6-ethoxypurine and comparing the size of the colonies that appear after culture under the same conditions as described above. For example, when a strain triply deficient *in purR, purA* and *deoD* derived from the *Bacillus subtilis* 168 Marburg strain was cultured at a 6-ethoxypurine content of 2000 mg/L in the medium, the relative growth degree calculated according to the aforementioned equation was about 40 to 60 with a culture time of 42 to 45 hours, or about 50 to 70 with a culture time of 48 to 66 hours, whereas the mutant strain obtained in the examples section showed a relative growth degree of about 80 to 100 with a culture time of 42 to 52 hours and the same content of 6-ethoxypurine. Therefore, by using the relative growth degree as an index, growth inhibition by 6-ethoxypurine can be evaluated without comparison with the parent strain. However, the growth inhibition by 6-ethoxypurine can also be evaluated by comparing the relative growth degrees of the parent strain and the mutant strain.

By culturing the *Bacillus* bacterium of the present invention obtained as described above in an appropriate medium, inosine can be produced and accumulated in the medium.

As for the medium used in the present invention, culture can be performed in a conventional manner using a typical medium containing a carbon source, nitrogen source and mineral salts as well as organic trace nutrients such as amino acids and vitamins, as required. Either a synthetic medium or a natural medium may be used. Any kind of carbon source and nitrogen source may be used so long as they can be utilized by the strain to be cultured.

As the carbon source, sugars such as glucose, glycerol, fructose, sucrose, maltose, mannose, galactose, starch hydrolysates and molasses can be used, and organic acids such as acetic acid and citric acid can also be used either alone or in combination with other carbon sources.

As the nitrogen source, ammonia, ammonium salts such as ammonium sulfate, ammonium carbonate, ammonium chloride, ammonium phosphate and ammonium acetate, nitric acid salts and so forth can be used.

As the organic trace nutrients, amino acids, vitamins, fatty acids, nucleic acids, those containing these substances such as peptone, casamino acid, yeast extract and soybean protein hydrolysate and so forth may be used. When an auxotrophic mutant strain that requires an amino acid or the like for its growth is used, it is necessary to supplement the required nutrient.

As the mineral salts, phosphoric acid salts, magnesium salts, calcium salts, ferrous salts, manganese salts and so forth can be used.

Although the culture condition may vary depending on the type of *Bacillus* bacterium to be used, *Bacillus subtilis,* for example, is cultured as aeration culture, while the fermentation temperature is controlled to be between 20 to 50°C, and pH to be between 4 to 9. When pH falls during the culture, the medium is neutralized with an alkali such as ammonia gas. Inosine is accumulated in the culture broth after 40 hours to 3 days of culture in a manner as described above.

After completion of the culture, inosine which has accumulated in the culture broth may be collected in a conventional manner. For example, inosine can be isolated by precipitation, ion exchange chromatography and so forth.

Furthermore, if the microorganism used for the present invention is made deficient in a gene coding for a nucleosidase or nucleotidase, a corresponding nucleoside or nucleotide can be accumulated. Furthermore, if inosine auxotrophy is imparted, a precursor or relevant substances involved in the biosynthesis pathway thereof can be accumulated.

Furthermore, by allowing purine nucleoside phosphorylase and/or phosphoribosyltransferase to act on inosine obtained by the method of the present invention, 5'-inosinic acid can be obtained.

Examples

Hereinafter, the present invention will be explained more specifically with reference to the following non-limiting examples.

Example 1

A recombinant which is derived from *Bacillus subtilis (B. subtilis* 168 Marburg strain, ATCC 6051) and which is deficient in the purine operon repressor gene *(purR),* succinyl-AMP synthase gene *(purA)* and purine nucleoside phosphorylase gene *(deoD)* was prepared as follows.

(1) Acquisition of purine operon repressor gene *(purR)* deficient strain

Based on information of a gene data bank (GenBank Accession No. Z99104), primers for PCR having the following nucleotide sequences, each of which was a 28-mer, were prepared.

CTCAAGCTTGAAGTTGCGATGATCAAAA (SEQ ID NO: 1)

CTCCTGCAGACATATTGTTGACGATAAT (SEQ ID NO: 2)

The chromosomal DNA of the *B. subtilis* 168 Marburg strain was used as a template together with the aforementioned primers to perform PCR (a cycle consisting of 94°C for 30 second, 55°C for 1 minute, and 72°C for 1 minute was repeated for 30 cycles using Gene Amp PCR System Model 9600 (Perkin-Elmer)) to obtain an amplified fragment of about 0.9 kb of the *purR* gene region covering SD-ATG and the translation termination codon.

On the 5' -end of the aforementioned primers for PCR, a *Hin*dIII site and *Pst*I site were designed, respectively. The PCR-amplified fragment was treated with *Hin*dIII and *Pst*I and then ligated to the pHSG398 vector (Takara Shuzo) digested with the same restriction enzymes, which was replicable in *E. coli,* by using T4 DNA ligase, to obtain a pHSG398BSPR plasmid.

An internal sequence of about 0.3 kb in the *purR* structural gene existing between the *Eco*RV and *Hin*cII sites, each of which is unique in the *purR* structural gene, was removed from pHSG398BSPR plasmid by a treatment with *Eco*RV and *Hin*cII. Then, the spectinomycin resistance gene (1.2 kb) derived from *Enterococcus faecalis* and cloned in pDG1726 plasmid (which can be prepared from the *Escherichia coli* ECE101 strain commercially available from the Bacillus Genetic Stock Center (The Ohio State University, Department of Biochemistry (484 West Twelfth Avenue, Columbus, Ohio 43210 USA))) was excised as an *EcoRV-Hinc*II fragment and inserted between the aforementioned restriction enzyme sites of pHSG398BSPR.

The resulting plasmid pHSG398purR::spc was used to transform competent cells of the *B. subtilis* 168 Marburg strain prepared according to the method of Dubunau and Davidoff-Abelson (J. Mol. Biol., 56, 209 (1971)), and the colonies grown on an LB (10 g/L of trypton, 5 g/L of yeast extract, 10 g/L of NaCl, pH 7) agar plate containing 100μg/ml of spectinomycin were selected. Chromosomal DNAs were prepared from these colonies, and a strain in which the *purR* gene on a chromosome was replaced with the *purR* gene of which internal sequence was replaced with the spectinomycin resistance gene (*purR*::spc) by double cross over recombination was identified by the aforementioned PCR method. One of the recombinant strains obtained as described above was designated as KMBS4.

(2) Acquisition of succinyl-AMP synthase gene *(purA)* deficient strain

Based on information of a gene data bank (GenBank Accession No. Z99104), primers for PCR having the following nucleotide sequences, each of which was a 29-mer, were prepared.

CTCGTCGACAAAACGAATGGAAGCGAACG (SEQ ID NO: 3)

CTCGCATGCAGACCAACTTATATGCGGCT (SEQ ID NO: 4)

The chromosomal DNA of the *B. subtilis* 168 Marburg strain was used as a template together with the aforementioned primers to perform PCR (a cycle consisting of 94°C for 30 second, 55°C for 1 minute, and 72°C for 2 minute was repeated for 30 cycles using Gene Amp PCR System Model 9600 (Perkin-Elmer)) to obtain an amplified fragment of about 0.9 kb of the *purA* gene region covering SD-ATG and the translation termination codon.
On the 5'end of the aforementioned primers for PCR, an *Sal*I site and *Sph*I site were designed, respectively. The PCR-amplified fragment was treated with *Sal*I and *Sph*I and then ligated to the pSTV28 vector (Takara Shuzo) digested with the same restriction enzymes, which was replicable in *E. coli,* using T4 DNA ligase, to obtain a pSTV28BSPA plasmid.
An internal sequence of about 0.4 kb in the *purA* structural gene existing between the *Mlu*I site and *Bgl*II site, each of which is unique in the *purA* structural gene, was removed from pSTV28BSPA plasmid by a treatment with *Mlu*I and *Bgl*II*,* and the both ends of the DNA fragment containing the plasmid structure were blunt-ended using the Klenow fragment. To this DNA fragment, an erythromycin resistance gene (1.6 kb) derived from *Staphylococcus aureus* and cloned on the pDG646 plasmid (which can be prepared from the *Escherichia coli* ECE91 strain commercially available from the Bacillus Genetic Stock Center) was ligated using T4 DNA ligase, after it was excised from the pDG646 plasmid by a treatment with *Hind*III, and both ends were blunt-ended using the Klenow fragment.
The obtained plasmid pSTV28purA::erm was used to transform competent cells of the KMBS4 strain prepared according to the method of Dubunau and Davidoff-Abelson (J. Mol. Biol., 56, 209 (1971)), and the colonies grown on an LB agar plate containing 0.1 µMg/ml of erythromycin and 125µg/ml of lincomycin. Chromosomal DNAs were prepared from these colonies, and strains (purR::spc, *purA::erm)* in which the *purA* gene on a chromosome was replaced with the *purA* gene of which internal sequence was replaced with the erythromycin resistance gene *(purA::erm)* by double cross over recombination were identified by the aforementioned PCR method. The recombinant strains obtained as described above became adenine auxotrophic strains, and one of them was designated as KMBS13.

(3) Acquisition of purine nucleoside phosphorylase gene *(deoD)* deficient strain

(i) Cloning of the 5' -end region of *deoD*

Based on information of a gene data bank (GenBank Accession No. Z99104), primers for PCR having the following nucleotide sequences, which were a 29-mer and 28-mer, respectively, were prepared.

CTCGAATTCCAGCGGAATATTCTTTCCCG (SEQ ID NO: 5)

CTCGGATCCCGGCAAAAGCACAGTATCC (SEQ ID NO: 6)

The chromosomal DNA of the *B. subtilis* 168 Marburg strain was used as a template together with the aforementioned primers to perform PCR (a cycle consisting of 94°C for 30 second, 55°C for 1 minute and 72°C for 1 minute was repeated for 30 cycles using Gene Amp PCR System Model 9600 (Perkin-Elmer)) to obtain an amplified fragment containing about 310 bp upstream from the *deoD* gene translation initiation codon and about 60 bp downstream from the same.
On the 5'-end of the aforementioned primers for PCR, an *Eco*RI site and *Bam*HI site were designed, respectively. The PCR-amplified fragment was treated with *Eco*RI and *Bam*HI and then ligated to the pSTV28 vector (Takara Shuzo) digested with the same restriction enzymes by using T4 DNA ligase to obtain a pSTV28DON plasmid.

(ii) Cloning of the 3'-end region of *deoD*

Based on information of a gene data bank (GenBank Accession No. Z99104), primers for PCR having the following nucleotide sequences, which were a 29-mer and 28-mer, respectively, were prepared.

CTCAAGCTTATGGTTTCCAGACCATCGACT (SEQ ID NO: 7)

CTCGGATCCCATGATATGATAGAAGTGG (SEQ ID NO: 8)

The chromosomal DNA of the *B. subtilis* 168 Marburg strain was used as a template together with the aforementioned primers to perform PCR (a cycle consisting of 94°C for 30 second, 55°C for 1 minute and 72°C for 1 minute was repeated for 30 cycles using Gene Amp PCR System Model 9600 (Perkin-Elmer)) to obtain an amplified fragment containing about 40 bp upstream from the *deoD* gene translation termination codon and about 320 bp downstream from the same.

On the 5'-end sides of the aforementioned primers for PCR, a *Hin*dIII site and *Bam*HI site were designed, respectively. The PCR-amplified fragment was treated with *Hin*dIII and *Bam*HI and then ligated to the pSTV28DON plasmid digested with the same restriction enzymes using T4 DNA ligase to obtain a pSTV28DONC plasmid.

(iii) Insertion of kanamycin resistance gene into *deoD*

Based on information of a gene data bank (GenBank Accession No. V01547), primers for PCR having the following nucleotide sequences, each of which was a 33-mer, were prepared.

CTCGGATCCGAGGTGATAGGTAAGATTATACCG (SEQ ID NO: 9)

CTCGGATCCGGCGCTCGGGACCCCTATCTAGCG (SEQ ID NO: 10)

The pDG783 plasmid containing the kanamycin resistance gene derived from *Streptococcus faecalis* (Bacillus Genetic Stock Center) was used as a template together with the aforementioned primers to perform PCR (a cycle consisting of 94°C for 30 second, 55°C for 1 minute and 72°C for 1 minute was repeated for 30 cycles using Gene Amp PCR System Model 9600 (Perkin-Elmer)) to obtain an amplified fragment having about 1.5 kb of the kanamycin resistance gene region covering SD-ATG and the translation termination codon.

On each of the 5'-ends of the primers for PCR, a *Bam*HI site was designed. The PCR-amplified fragment was treated with *Bam*HI and inserted into the pSTV28DONC plasmid at the *Bam*HI site, which was unique in the plasmid.

The resulting plasmid pSTV28deoD::kan was used to transform competent cells of the KMBS13 strain prepared according to the method of Dubunau and Davidoff-Abelson (J. Mol. Biol., 56, 209 (1971)), and the colonies grown on an LB agar plate containing 5 μg/ml of kanamycin. Chromosomal DNAs were prepared from these colonies, and strains in which the *deoD* gene on a chromosome was replaced with the *deoD* gene of which internal sequence was replaced with the kanamycin resistance gene (deoD::kan) by two times of recombination (purR::spc purA::erm deoD::kan) were identified by the aforementioned PCR method. One of the double recombinant strains obtained as described above was designated as KMBS16.

Example 2: Selection of 6-ethoxypurine-resistant strain

(1) Screening of 6-ethoxypurine resistant strain

The *Bacillus subtilis* recombinant strain KMBS16 deficient in the purine operon repressor gene *(purR),* succinyl-AMP synthase gene *(purA)* and purine nucleoside phosphorylase gene *(deoD)* was treated with 400μg/ml of nitrosoguanidine (NTG) at 37°C for 30 minutes. Then, the NTG-treated cell suspension was spread on a minimal medium (20 g/L of glucose, 5 g/L of ammonium chloride, 4 g/L of potassium dihydrogenphosphate, 0.01 g/L of ferrous sulfate, 0.01 g/L of manganese sulfate, 0.5 g/L of sodium citrate, 1.0 g/L of L-glutamic acid, 0.1 g/L of adenine, 50 mg/L of tryptophan, pH 7.0) plate containing 2000 mg/L of 6-ethoxypurine, and the cells were cultured at 37°C for 24 hours. Among the colonies exhibiting favorable growth on the plate containing 2000 mg/L of 6-ethoxypurine, ten strains were selected and designated as EP1 to EP10 strains. The EP1 strain was given a private number of AJ13937. This strain was deposited at the independent administrative agency, National Institute of Advanced Industrial Science and Technology, International Patent Organism Depositary (Tsukuba Central 6, 1-1, Higashi 1-Chome, Tsukuba-shi, Ibaraki-ken, 305-8566, Japan) on December 25, 2001 and received an accession number of FERM P-18665. Then, it was transferred to an international deposit under the provisions of the Budapest Treaty on January 14, 2004, and received an accession number of FERM BP-08595.

When resistance of the ethoxypurine-resistant strain obtained as described above to various drugs conventionally used for the breeding of known inosine producing bacteria was investigated, it exhibited a tendency similar to that of the parent strain.

(2) Assay for degree of 6-ethoxypurine resistance

Degree of 6-ethoxypurine resistance of the mutant strain obtained as described above was examined in detail. The parent strain and the mutant strain were adenine auxotrophic, and the drug used was a purine analogue. Therefore, the amount of adenine added to the minimal medium was limited to the required minimum level (2 mg/L in this example).

The 6-ethoxypurine-resistant strains (EP1 strain and EP2 strain) and the parent strain KMBS16 were each inoculated into the minimum liquid medium and cultured overnight at 34°C. The cells were separated from the culture broth by centrifugation, washed three times with the minimal medium and suspended again in the same medium. The suspension was appropriately diluted with the same medium and spread on a minimal medium plate and a minimal medium plate containing 2000 mg/L of 6-ethoxypurine, and the cells were cultured at 34°C. After 24 hours, minute colonies were confirmed for the EP1 strain and EP2 strain, whereas no colony was confirmed for the parent strain KMBS16. The culture was continued even thereafter, and diameters of several single colonies were measured after 48 hours to 66 hours. An average of colony diameters was calculated for each strain, and the relative growth degree was calculated according to the aforementioned equation.

The diameters of colonies measured as described above and relative growth degrees are shown in Tables 1 to 3. In the tables, the symbol "-" means that no colony could be confirmed, and the symbol "±" means that since the colonies were indefinite, the diameters could not be measured.

Table 1:

| Diameter of colony on minimal medium plate not containing 6-ethoxypurine | | | | | | | |
|---|---|---|---|---|---|---|---|
| Strain NO. | Diameter of colony (mm) | | | | | | |
| | | 28hr | 42hr | 45hr | 48hr | 52hr | 66hr |
| KMBS16 | 1 | ± | 1 | 1.2 | 1.5 | 1.5 | 2.0 |
| EP1 | 1 | ± | 1 | 1.2 | 1.5 | 1.5 | 2.0 |

Table 2:

| Diameter of colony on minimal medium plate containing 6-ethoxypurine (2000μg/mL) | | | | | | | |
|---|---|---|---|---|---|---|---|
| Strain NO. | Diameter of colony (mm) | | | | | | |
| | | 28hr | 42hr | 45hr | 48hr | 52hr | 66hr |
| KMBS16 | 1 | - | 0.50 | 0.70 | 1.00 | 1.00 | 1.25 |
| | 2 | - | 0.40 | 0.50 | 0.75 | 1.00 | 1.00 |
| EP1 | 1 | ± | 0.80 | 1.00 | 1.20 | 1.50 | 2.50 |
| | 2 | ± | 1.00 | 1.20 | 1.50 | 1.50 | 2.00 |
| EP2 | 1 | ± | 1.00 | 1.20 | 1.50 | 1.50 | 2.00 |
| | 2 | ± | 1.00 | 1.20 | 1.50 | 1.50 | 2.00 |

Table 3:

| Relative growth degree | | | | | | | | |
|---|---|---|---|---|---|---|---|---|
| Strain NO. | | Relative growth degree (%) | | | | | | |
| | | 0hr | 42hr | 45hr | 48hr | 52hr | 66hr |
| KMBS16 | 1 | 0 | 50.0 | 58.3 | 66.7 | 66.7 | 62.5 |
| | 2 | 0 | 40.0 | 41.7 | 50.0 | 66.7 | 50.0 |
| EP1 | 1 | 0 | 80.0 | 83.3 | 80.0 | 100.0 | |
| | 2 | 0 | 100.0 | | | | |
| EP2 | 1 | 0 | 100.0 | | | | |
| | 2 | 0 | 100.0 | | | | |

Example 3: Production of purine nucleic acids by 6-ethoxypurine-resistant strain

The 6-ethoxypurine-resistant strain (EP1 strain) and the parent strain KMBS16 were each uniformly applied on an LB medium plate and cultured overnight at 37°C. The cells of 1/6 of the plate were inoculated into 20 ml of fermentation medium in a 500-ml volume elemental flask, and after calcium carbonate was added to the medium at a concentration of 50 g/L, the cells were cultured at 37°C with shaking. After the start of the culture, the medium was sampled in the time course, and the amounts of inosine and hypoxanthine contained in the culture broth were measured using known methods. All of the 6-ethoxypurine-resistant strains exhibited higher inosine and hypoxanthine accumulations compared with the parent strain KMBS16. As representative examples, the results measured after 42 hours are shown (Fig. 1).

| [Composition of a fermentation medium] | |
|---|---|
| Glucose | 80 g/L |
| $KH_2PO_4$ | 1 g/L |
| $NH_4Cl$ | 32 g/L |
| Mameno (T-N)* | 1.35 g/L |
| DL-Methionine | 0.3 g/L |
| L-Tryptophan | 0.02 g/L |
| Adenine | 0.1 g/L |
| $MgSO_4$ | 0.4 g/L |
| $FeSO_4$ | 0.01 g/L |
| $MnSO_4$ | 0.01 g/L |
| GD113 | 0.01 ml/L |
| (adjusted to pH 7.0 with KOH) Calcium carbonate | 50 g/L |

*: Soybean protein hydrolysates
** Anti-foam-reagent

## Claims

1. A *Bacillus* bacterium which is modified so that growth inhibition by 6-ethoxypurine is reduced and has inosine-producing ability.

2. The *Bacillus* bacterium according to claim 1, which is a mutant strain derived from a parent strain belonging to the genus *Bacillus* and shows favorable growth as compared with the parent strain when cultured in a medium containing 6-ethoxypurine.

3. The *Bacillus* bacterium according to claim 2, wherein the medium has an ethoxypurine content of 2000 mg/L.

4. The *Bacillus* bacterium according to claim 1, wherein the medium is a solid medium.

5. The *Bacillus* bacterium according to claim 1, wherein when the bacterium is cultured by applying a suspension of the bacterium to a solid medium containing 6-ethoxypurine and a solid medium not containing 6-ethoxypurine, the bacterium shows a relative growth degree of 80 or more, which is defined by the following equation:

Relative growth degree (%) = [colony diameter (mm) observed in the medium containing 6-ethoxypurine]/[colony diameter (mm) observed in the medium not containing 6-ethoxypurine] x 100.

6. The *Bacillus* bacterium according to claim 5, wherein the solid medium containing 6-ethoxypurine has a 6-ethoxypurine content of 2000 mg/L.

7. The *Bacillus* bacterium according to claim 6, wherein the solid medium is a minimal medium.

8. The *Bacillus* bacterium according to claim 1, which is deficient in one or more genes negatively acting on the biosynthesis of inosine or involved in degradation of inosine and selected from a purine operon repressor gene, succinyl-AMP synthase gene and purine nucleoside phosphorylase gene.

9. A method for producing a *Bacillus* bacterium having improved inosine-producing ability, which comprises selecting strains showing favorable growth in a medium containing 6-ethoxypurine from a population of *Bacillus* bacteria, and selecting a strain showing high inosine-producing ability from the obtained strains.

10. The method according to claim 9, wherein the population of *Bacillus* bacteria is obtained by subjecting a parent strain belonging to the genus *Bacillus* to a mutagenesis treatment.

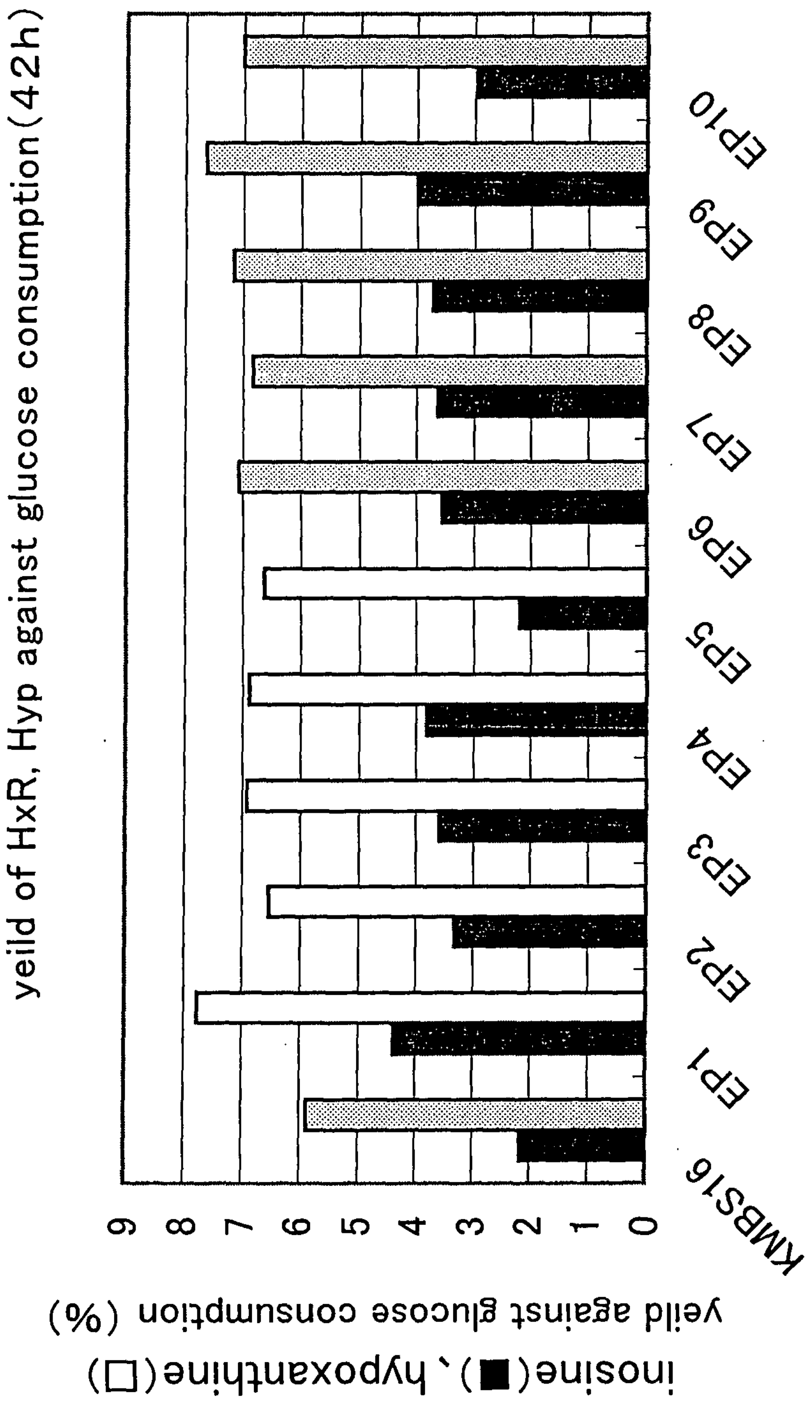
yeild of HxR, Hyp against glucose consumption (42h)
inosine(■), hypoxanthine(□)
yeild against glucose consumption (%)
0
1
2
3
4
5
6
7
8
9
KMBS16
EP1
EP2
EP3
EP4
EP5
EP6
EP7
EP8
EP9
EP10

Fig. 1

European Patent Office

# EUROPEAN SEARCH REPORT

Application Number
EP 04 00 3190

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 3 535 207 A (SHIRO TERUO ET AL) 20 October 1970 (1970-10-20) * abstract; column 1, lines 60-66; column 2, lines 26-59; examples 3 and 6 * ----- | 1,2,4,7 | C12N1/20 C12P19/40 |
| X | DATABASE WPI Section Ch, Week 197806 Derwent Publications Ltd., London, GB; Class B02, AN 1978-11074A XP002282914 & JP 52 154595 A (AJINOMOTO KK) 22 December 1977 (1977-12-22) * abstract * ----- | 1-10 | |
| X | ISHII K ET AL: "IMPROVED INOSINE PRODUCTION AND DE REPRESSION OF PURINE NUCLEOTIDE BIOSYNTHETIC ENZYMES IN 8 AZA GUANINE RESISTANT MUTANTS OF BACILLUS-SUBTILIS" AGRICULTURAL AND BIOLOGICAL CHEMISTRY, vol. 36, no. 9, 1972, pages 1511-1522, XP009031722 ISSN: 0002-1369 * abstract * ----- | 1-10 | TECHNICAL FIELDS SEARCHED (Int.Cl.7) C12N C12P |
| X | PATENT ABSTRACTS OF JAPAN vol. 0072, no. 81 (C-200), 15 December 1983 (1983-12-15) & JP 58 158197 A (AJINOMOTO KK), 20 September 1983 (1983-09-20) * abstract * ----- | 1-10 | |

The present search report has been drawn up for all claims

| Place of search | Date of completion of the search | Examiner |
|---|---|---|
| Munich | 2 June 2004 | Fausti, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 00 3190

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-2004

| Patent document cited in search report | Publication date | Patent family member(s) | Publication date |
|---|---|---|---|
| US 3535207 A | 20-10-1970 | FR 1514638 A | 23-02-1968 |
| | | GB 1127544 A | 18-09-1968 |
| JP 52154595 A | 22-12-1977 | JP 1229020 C | 19-09-1984 |
| | | JP 59004996 B | 02-02-1984 |
| JP 58158197 A | 20-09-1983 | JP 1668048 C | 29-05-1992 |
| | | JP 3033318 B | 16-05-1991 |

EPO FORM P0459

For more details about this annex : see Official Journal of the European Patent Office, No. 12/82